# EUROPEAN PATENT SPECIFICATION

(11) **EP 1 812 370 B1**
(45) Date of publication and mention of the grant of the patent: **25.06.2008**
(21) Application number: 05802376.3
(22) Date of filing: 11.11.2005
(51) Int. Cl.: C07C 45/74, C07C 49/597, C07C 49/657, C07C 49/203

(54) **SYNTHESIS OF CYCLOPENTENONES**
SYNTHESE VON CYCLOPENTENONEN
SYNTHESE DE CYCLOPENTENONES

(30) Priority: 11.11.2004 WO PCT/IB2004/003719; 18.11.2004 WO PCT/IB2004/003854
(43) Date of publication of application: 01.08.2007
(73) Proprietor: FIRMENICH SA, CH-1211 Geneva 8 (CH)
(72) Inventor: JACOBY, Denis, CH-1260 NYON (CH); KELLER, Fabrice, CH-1242 SATIGNY (CH)
(74) Representative: Carina, Riccardo Filippo
(86) International application number: PCT/IB2005/053717
(87) International publication number: WO 2006/051503

(56) References cited:
- YUKI TSUYOSHI ET AL: "Direct synthesis of polysubstituted cyclopentenones from ketones and aldehydes catalyzed by zirconium compounds" JOURNAL OF ORGANIC CHEMISTRY, vol. 58, no. 16, 1993, pages 4497-4499, XP002364567 ISSN: 0022-3263 cited in the application

## Description

### Technical field

The present invention relates to the field of organic synthesis and more precisely to a single step process for the synthesis of a cyclopentenone derivative of formula 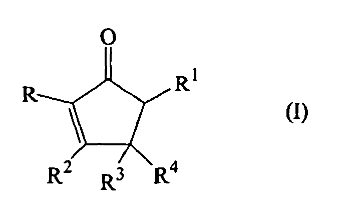 as defined further below.

### Prior art

Ishii et al., in J.Org.Chem., 1993, 58, 4497, reports the synthesis of cyclopentenone derivatives from the reaction of a ketone with two equivalents of an aldehyde or by the reaction of an enone with an aldehyde, which is catalyzed by Zirconium chloride derivatives such as ZrOCl₂ or ZrCl₄, the oxide being described as the best catalyst.

However, the procedure reported by Ishii requires severe conditions such as high temperature (between 130 and 200°C, the upper part of the range giving the best results, see Table I). Such severe conditions may result in poor yields (for example 17% when all the ring substituents are methyl groups, reaction carried out at 200°C). These conditions are not of high industrial interest since are not environmentally friendly, and require much energy and produce high amounts of wastes.

### Description of the invention

In order to overcome all or part of the problems aforementioned, the present invention relates to a process for the preparation of cyclopentenone derivatives that can be carried out with soft conditions and accessory can result in high yields.

One of the objects of the invention is a process for the preparation of a compound of formula 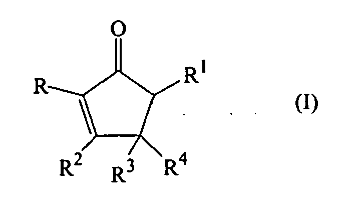 wherein R represents a C₁₋₈ alkyl or alkenyl group optionally substituted or a C₅₋₆ aromatic, optionally substituted; and
R¹, R², R³ and R⁴ represent, simultaneously or independently, a hydrogen atom, a C₁₋₈ alkyl or alkenyl group optionally substituted or a C₅₋₆ aromatic group, optionally substituted;
said process comprising the reaction between an enone of formula 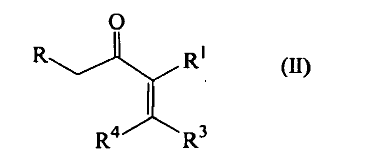 wherein R, R¹, R³ and R⁴ have the same meaning as in formula (I);
with an aldehyde of formula 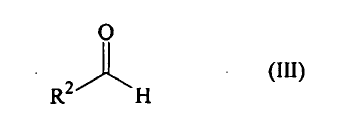 wherein R² has the same meaning as in formula (I); and
the reaction between said enone (II) and the aldehyde (III) being performed in the presence of a catalytic system comprising:
i) at least one metal complex of formula

   M(OR⁵)₄₋ₙXₙ (IV)

   wherein M is Ti(IV) or Zr(IV), R⁵ represents a C₁₋₆ linear or branched alkyl group, X represents a halide and n represents an integer from 1 to 3; and
ii) at least one co-ingredient selected from the group consisting of
   a) an alkyl or aromatic carboxylic acid anhydride containing 2 to 10 carbon atoms;
   b) an anhydrous sulfate, chloride or bromide of a metal cation selected from the group consisting of Li⁺, Na⁺, K⁺, Cs⁺, Mg²⁺, Ni²⁺, C²⁺ Zn²⁺, Fe³⁺ and Al³⁺;
   c) an insoluble inorganic material capable to form a clathrate with water; and
   d) a C₄-C₁₅ orthoester, BF₃, N-methyl-N-trimethylsilyl-trifluoroacetamide, 1-trimethylsilylimidazole and ClSi(R⁶)₃, R⁶ representing a C₁₋₅ alkyl group.

Possible optional substituents of said groups R, R¹, R², R³ and R⁴ are groups which do not affect the reactivity of the enone (II) or of the aldehyde (III). Examples of said optional substituents, when said R, R¹, R², R³ and R⁴ groups represent alkyl or alkenyl group, include one or two methyl, ethyl, methoxy or ethoxy groups. Examples of said optional substituents, when said R, R¹, R², R³ and R⁴ groups represent an aromatic group, include one or two methyl, ethyl, methoxy, ethoxy or nitro groups.

According to a particular embodiment of the invention, R represents a C₁₋₈ alkyl or alkenyl group or C₅₋₆ aromatic group optionally substituted. Alternatively, R¹, R², R³ and R⁴ represent, simultaneously or independently, a hydrogen atom, a C₁₋₈ alkyl or alkenyl group or a C₅₋₆ aromatic group optionally substituted.

In particular according to said embodiment, R represents a C₁₋₈ alkyl or alkenyl group, and R¹, R², R³ and R⁴ represent, simultaneously or independently, a hydrogen atom, a C₁₋₈ alkyl or alkenyl group.

In another embodiment of the invention, there is obtained an enone of formula (I), from the corresponding compounds (II) and (III), wherein R represents a methyl, ethyl or pentyl group or a phenyl group optionally substituted. Alternatively, or simultaneously, R¹, R², R³ and R⁴ represent, simultaneously or independently, a hydrogen atom, a methyl, ethyl or pentyl group or a phenyl group optionally substituted.

In particular according to said embodiment, R represents a methyl, ethyl or pentyl group, and R¹, R², R³ and R⁴ represent, simultaneously or independently, a hydrogen atom, a methyl, ethyl or pentyl group.

According to any one of the above-mentioned embodiments, R⁴ represents a hydrogen atom. In particular, said R, R¹, R² or R³ represents, simultaneously or independently, a methyl, ethyl or phenyl group optionally substituted, or just a methyl or ethyl group, while R⁴ represents a hydrogen atom.

According to a further embodiment of the invention, the ketone (II) can be obtained *in situ* by reacting together a ketone (V) and a ketone or aldehyde (VI), in the presence of the same catalytic system of the invention's process, according to Scheme 1. 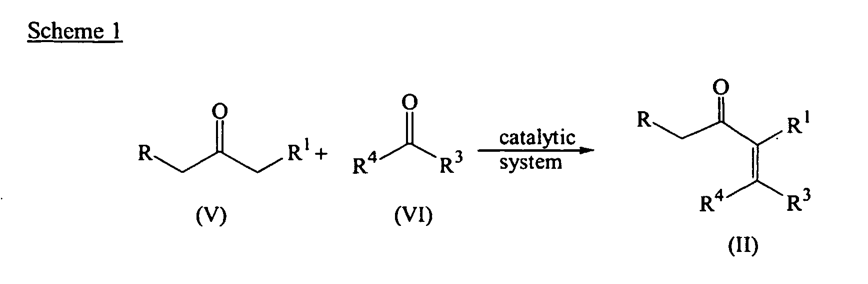 wherein R, R¹, R³ and R⁴ have the same meaning as indicated above.

Therefore the present invention concerns also a process further comprising the step mentioned above.

Particular examples of suitable ketones (II), (V) or (VI) are diethyl ketone, dibenzyl ketone, methyl phenyl ketone, ethyl phenyl ketone or ⁿhexyl methyl ketone.

Particular examples of suitable aldehydes (III) or (VI) are acetaldehyde, formaldehyde, propanaldehyde or benzaldehyde.

According to a further embodiment of the invention, the process comprises a first step wherein are reacted together a diethyl ketone and acetaldehyde to obtain a ketone of formula (II), which is subsequently reacted with acetaldehyde.

According to an embodiment of the present invention the molar ratio between the enone (II) and the aldehyde (III) is comprised between 1.1/1 and 1/6, more preferably between 1/1 and 1/5 and even more preferably between 1/1.1 and 1/5. Furthermore, the molar ratio between the ketone (V) and the compound (VI) is comprised between 1/1 and 1/8, more preferably between 1/2.5 and 1/6 and even more preferably between 1/3 and 1/5.

As mentioned above, the process of the invention is carried out in the presence of a catalytic system, which consists of a metal complex and of a co-ingredient. The metal complex is used in substoechiometric, or catalytic amounts, relative to the starting aldehyde or ketone.

The metal complex has a general formula:

M(OR⁵)₄₋ₙXₙ (IV)

wherein M, n, R⁵ and X have the meaning given above. According to a particular embodiment of the invention, M represents Ti(IV), R⁵ represents a linear or branched C₃₋₄ alkyl group, X represents a Cl atom and the index n represents 2 or 3.

The use of a mixture of metal complexes of formula (IV) is also convenient, especially if the catalyst is synthesized *in situ,* and without purification, prior to its use in the process.

According to a particular embodiment of the invention, the co-ingredient of the catalytic system is selected from the group consisting of an alkyl or aromatic carboxylic acid anhydride containing 4 to 8 carbon atoms, BF₃, ClSi(R⁶)₃, R⁶ representing a C₁₋₅ alkyl group, and an anhydrous sulfate, chloride or bromide of a metal cation selected from the group consisting of Na⁺, + K⁺, Mg²⁺, Ca²⁺, Zn²⁺, Fe³⁺.

Preferably, the co-ingredient is selected from the group consisting of acetic, propionic or butyric anhydride, BF₃, ClSi(R⁶)₃, R⁶ representing a methyl or ethyl group. the anhydrous Na₂SO₄ or K₂SO₄ and an anhydrous chloride or bromide of Mg²⁺, Fe³⁺ or Zn²⁺.

The use of a mixture of two or three co-ingredients is also possible.

The metal complex can be added to the reaction medium in a large range of concentrations. As non-limiting examples, one can cite catalyst concentrations ranging from 0.01 to 0.20 molar equivalents, relative to the molar amount of the starting ketone (II) or (V). Preferably, the metal complex concentration will be comprised between 0.01 and 0.10 molar equivalents. It goes without saying that the optimum concentration of catalyst will depend on the nature of the latter and on the desired reaction time.

The co-ingredient can be added to the reaction medium in a large range of concentrations. As non-limiting examples, one can cite salt concentrations ranging from 0.05 to 1.2 molar equivalents, relative to the number of moles of the starting ketone (II) or (V). Preferably, the salt concentration will be comprised between 0.10 and 0.60 molar equivalent. Yet, in another preferred embodiment, the salt concentration will be comprised between 0.20 and 0.50 molar equivalents. It goes without saying that the optimum concentration of the additional agent will depend on the nature of the latter.

The process of the invention can be carried out in the presence or absence of solvent, but in any case it is advantageously performed in anhydrous conditions, wherein by "anhydrous" it is meant here a solvent which has a content in water below 1% by weight, preferably below 0.1 %. When a solvent is required, it is possible to use a pure solvent or a mixture of solvents. Said solvent must be chemically compatible with the reaction conditions, i.e. not interfere with the reaction, and not deactivate the catalyst, e.g. a weak or non-coordinating solvent. Preferred solvents for the process of the invention have a boiling point higher than 60°C and are selected from the group consisting of ethers, esters, aromatic solvents, and linear or branched or cyclic hydrocarbons. More preferably, the solvent is an ester such as butyl acetate.

Furthermore, the solvent can be the starting ketone (II) or (V) or the starting aldehyde (III) or (VI).

The temperature at which the process of the invention can be carried out is comprised between 60°C and 140°C, preferably between 70°C and 100 or 110°C. Of course a person skilled in the art is also able to select the reaction temperature as a function of the melting and boiling point of the starting and final products and/or the possible solvent.

The invention will now be described in further detail by way of the following examples, the temperatures are indicated in degrees centigrade (°C); the NMR spectral data were recorded with a 360MHz machine in CDCl₃, the chemical displacement δ are indicated in ppm with respect to the TMS as standard, the coupling constant J are expressed in Hz and all the abbreviations have the usual meaning in the art.

### Example 1

### Synthesis of 2,3,4,5-tetramethyl-2-cyclopenten-1-one

### a) Preparation of the metal catalyst solution

A catalytic solution containing the TiCl₃(OⁱPr) complex is obtained according to the procedure described in E.V. Vedejs et al., J. Org. Chem., (1988), 53, 1593 but using the TiCl₄ and the Ti(OⁱPr)₄ complexes as starting materials. The quantities were modified in order to obtain catalytic solution with a concentration of 1.3 mmole of metal per gram of catalytic solution.
All the resulting solutions were used without further manipulation.

### b) Preparation of 2,3,4,5-tetramethyl-2-cyclopenten-1-one

In a bottom round flask equipped with a mechanical stirrer, a dropping funnel and a reflux condenser was loaded 2000g (23.2 mol) of the starting ketone with 75% w/w of butylacetate as the solvent, 0.35 molar equivalents of anhydrous magnesium chloride and the aforementioned titanium catalytic solution containing 0.06 molar equivalents of the trichloropropoxytitanium complex. The resulting suspension was stirred vigorously and allowed to heat to 90°C. Then 2 molar equivalents of the acetaldehyde were added dropwise over 3h at 90°C. The reaction was continued for an additional hour and cooled to 40°C. The reaction mixture was hydrolysed with a 10% aqueous acetic acid solution and neutralised with a 20% aqueous potassium carbonate solution.
The resulting organic phase was directly fractionated into a laboratory Sulzer packed column, to afford the title compound, as a mixture of isomers trans:cis = 85:15, in 27 % yield (B.p. = 70-80°C at P = 8 mbar) and the enone (II), (i.e. 4-methyl-4-hexen-3-one) in 31 % yield (B.p. = 45-65°C at P = 8 mbar).
¹H-NMR (isomer trans): 1.15 (d 3H); 1.19 (d 3H); 1.68 (s 3H); 1.88 (m 1H); 1.98 (s 3H); 2.25 (m 1H).
¹³C-NMR (isomer trans): 8.5; 14.6; 15.1; 17.7; 46.2; 48.4; 134.5; 171.6; 211.0.

### Example 2

### Synthesis of 3,4-diethyl-2,5-dimethyl-2-cyclopenten-1-one

In a bottom round flask equipped with a mechanical stirrer, a dropping funnel and a reflux condenser were loaded 265g (3.08 mol) of the diethyl ketone with 252 g of butylacetate as the solvent, 0.36 molar equivalents of anhydrous magnesium chloride and the aforementioned titanium catalytic solution containing 0.053 molar equivalents of the trichloropropoxytitanium complex. The resulting suspension was stirred vigorously and allowed to heat to 85°C. Then 2.1 molar equivalents of propionaldehyde were added dropwise over 2h at 85°C. The reaction was continued for an additional hour and cooled to 40°C. The reaction mixture was hydrolyzed with a 10% aqueous acetic acid solution (500g), decanted and washed with a 10% aqueous acetic acid solution (200g) and 50 g of NaCl. The organic phase was then washed twice with a 20% aqueous potassium carbonate solution.
After drying over Na₂SO4, the solvent was evaporated. The crude product (419.6 g), was distilled through a Vigreux column and then fractionated through a Fischer column, to afford the title compound in 17% yield (B.p. = 58°C at P = 3 mbar) and the enone (II) (i.e. 4-methyl-4-hepten-3-one) in 32 % yield (B.p. = 84°C at P = 43 mbar).
MS: 166 (76); 151 (37); 138 (100); 137 (94); 109 (72); 67 (54); 41 (30).
¹H-NMR: 2.52 (1H; m); 2.29 (2H; m); 2.01(1H; dq; 2.5 Hz; 8 Hz); 1.90-1.80 (1H; m); 1.69 (3H; br s); 1.34-1.22 (1H; m); 1.17 (3H; d; J=6 Hz); 1.10 (3H; d; J=7 Hz); 0.94 (3H; d; J=8 Hz).
¹³C-NMR: 211.8 (s); 175.9 (s); 134.4 (s); 50.5 (d); 45.2 (d); 25.2( t); 21.8 (t); 16.9 (q), 11.8 (q); 11.4 (q); 8.0 (q).

### Example 3

### Synthesis of various compounds of formula (I)

### General procedure for cyclopentenone process

In a bottom round flask equipped with a mechanical stirrer, a dropping funnel and a reflux condenser was loaded 1 molar equivalents of the starting ketone (see further below) neat or with 75% w/w of butylacetate as the solvent, 0.35 molar equivalents of anhydrous magnesium chloride and the aforementioned titanium catalytic solution containing 0.05 molar equivalents of the trichloropropoxytitanium complex. The resulting suspension was stirred vigorously and allowed to heat to 90-100°C. Then the aldehyde of formula (III) (see further below) was dropped over 3 hours at 90-100°C. The reaction was continued an additional hour at 90-100°C and cooled to 40°C. The reaction mixture was hydrolyzed with a 10% hydrochloric acid solution and neutralized with a 20% potassium carbonate solution.
The resulting organic phase was directly fractionated into a laboratory Sulzer packed column. The results are summarized herein below.

### Experiment A)

### Starting ketone: 4-methyl-4-hexen-3-one

### Starting aldehyde: benzaldehyde (1.2 molar equivalents)

### Products:

4-phenyl-2,3,5-trimethyl-2-cyclopenten-1-one + 3-phenyl-2,4,5-trimethyl-2-cyclopenten-1-one (20/80)
yield: 25% (based on used starting ketone)
yield: 45% (based on converted starting ketone)
Analysis for the major product (3-phenyl-2,4,5-trimethyl-2-cyclopenten-1-one):
¹H-NMR: 1.08 (d, 3H); 1.25 (d, 3H); 1.88 (s, 3H); 2.05 (m, 1H); 2.85 (m, 1H); 7.3-7.5 (m, 5H).
¹³C-NMR: 9.5; 15.5; 19; 45; 48.5; 127-129 (6C); 135; 170.5; 211.

### Experiment B)

### Starting ketone: 4-methyl-4-hexen-3-one

### Starting aldehyde: 10-undecenal (0.9 molar equivalents)

### Products:

4-(9-decenyl)-2,3,5-trimethyl-2-eyclopenten-1-one + 3-(9-decenyl)-2,4,5-trimethyl-2-cyclopenten-1-one (33/66)
yield: 36% (based on used starting ketone)
yield: 72% (based on converted starting ketone)
Analysis of the mixture obtained:
¹H-NMR: 1.15 (d); 1.17 (d); 1.25-1.40 (m); 1.70 (s); 1.90 (m); 2.0 (s); 2.05 (m); 2.35 (m); 2.50 (m); 4.95 (m, 2H); 5.80 (m, 1H).
¹³C-NMR: 8; 15; 17; 18; 27; 28-30; 33; 34; 44; 47; 49; 52; 114; 135; 135.5; 139; 171; 175.5; 211.

### Experiment C)

### Starting ketone: 5-ethyl-4-methyl-4-hepten-3-one

### Starting aldehyde: acetaldehyde (1.2 molar equivalents)

### Products:

4,4-diethyl-2,3,5-trimethyl-2-cyclopenten-1-one
yield: 40% (based on used starting ketone)
yield: 58% (based on converted starting ketone)
Analysis of the product:
¹H-NMR: 0.45 (t, 3H); 0.78 (t, 3H); 1.08 (d, 3H); 1.35-1.70 (m, 4H); 1.70 (s, 3H); 1.85 (s, 3H); 2.20 (q, 1H).
¹³C-NMR: 7.95; 8.75; 9.60; 10.10; 12.30; 27.55; 29.95; 46.40; 51.85; 136.3; 171.1; 210.3.

### Experiment D)

### Starting ketone: Diethylketone

### Starting aldehyde: Benzaldehyde (3.0 molar equivalents)

### Products:

3,4-diphenyl-2,5-dimethyl-2-cyclopenten-1-one cis/trans: 15/85
yield: 32% (based on used starting ketone)
yield of ketone (II): 24% (based on used starting ketone)
Analysis of the product:
*Trans isomer*
¹H-NMR: 1.34 (d, J=7.17, 3H); 2.02 (s, 3H); 2.40 (dq, J₁=7.17, J₂= 2.56, 1H);3.97 (sb, 1H); 7.0-7.3 (m 10H)
¹³C-NMR: 10.1; 15.3; 51.25; 56.33; 126-129 (10 CH); 135.2 ; 136.7; 142 ; 167; 210.9
*Cis isomer*
¹H-NMR: 0.75 (d, J=7.68, 3H); 2.08 (s, 3H); 2.92 (m, 1H); 4.6 (d, J=6.14, 1H); 7.0-7.3 (m, 10H)
¹³C-NMR: 9.8; 12.3; 45.5; 52.5; 126-129 (10 CH); 135.7 ; 136.9; 139.2 ; 166.3; 211.4

### Experiment E)

### Starting ketone: 1-3 diphenylacetone

### Starting aldehyde: acetaldehyde (3.0 molar equivalents)

### Products:

3,4-dimethyl-2,5-diphenyl-2-cyclopenten-1-one cis/trans: 15/85
yield: 48% (based on used starting ketone)
yield of ketone (II): 36% (based on used starting ketone)
Analysis of the product:
*Trans isomer*
¹H-NMR: 1.35 (d, J=6.65, 3H); 2.17 (s, 3H); 2.87 (dq, J₁=6.65, J₂= 3.07, 1H); 3.23 (d, J=3.07, 1H); 7.1-7.4 (m, 10H)
¹³C-NMR: 15.9; 18.1; 47.7; 60.7; 126-129 (10 CH); 131.8 ; 138.9; 139.5 ; 174; 205.
*Cis isomer*
¹H-NMR: 0.8 (d, J=7.17, 3H); 2.15 (s, 3H); 3.15 (m, 1H); 3.95 (d, J=7.17, 1H); 7.1-7.4 (m, 10H).
¹³C-NMR: 16.1; 16.3; 42.8; 56.7; 126-129 (11 CH); 137.7; 139.7 ; 174.7; 206.5

## Claims

1. A process for the preparation of a compound of formula 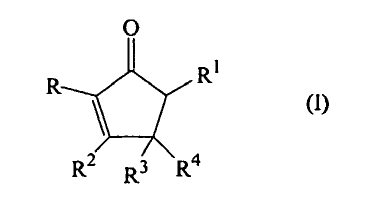 wherein R represents a C₁₋₈ alkyl or alkenyl group optionally substituted or a C₅₋₆ aromatic, optionally substituted; and
R¹, R², R³ and R⁴ represent, simultaneously or independently, a hydrogen atom, a C₁₋₈ alkyl or alkenyl group optionally substituted or a C₅₋₆ aromatic group, optionally substituted;
said process comprising the reaction between an enone of formula 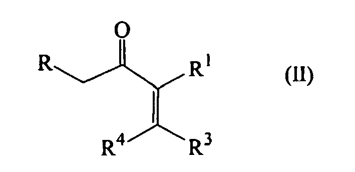 wherein R, R¹, R³ and R⁴ have the same meaning as in formula (I);
with an aldehyde of formula 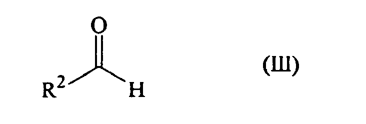 wherein R² has the same meaning as in formula (I); and
the reaction between said enone (II) and the aldehyde (III) being performed in the presence of a catalytic system comprising:
i) at least one metal complex of formula
M(OR⁵)₄₋ₙXₙ (IV)
wherein M is Ti(IV) or Zr(IV), R⁵ represents a C₁₋₆ linear or branched alkyl group, X represents a halide and n represents an integer from 1 to 3; and
ii) at least one co-ingredient selected from the group consisting of
a) an alkyl or aromatic carboxylic acid anhydride containing 2 to 10 carbon atoms;
b) an anhydrous sulfate, chloride or bromide of a metal cation selected from the group consisting of Li⁺, Na⁺, K⁺, Cs⁺, Mg²⁺, Ni²⁺, Ca²⁺, Zn²⁺, Fe³⁺ and Al³⁺;
c) an insoluble inorganic material capable to form a chlatrate with water; and
d) a C₄-C₁₅ orthoester, BF₃, N-methyl-N-trimethylsilyl-trifluoroacetamide, 1-trimethylsilylimidazole and ClSi(R⁶)₃, R⁶ representing a C₁₋₅ alkyl group.

2. A process according to claim 1, **characterized in that** R represents a C₁₋₈ alkyl or alkenyl group; and
R¹, R², R³ and R⁴ represent, simultaneously or independently, a hydrogen atom, a C₁₋₈ alkyl or alkenyl group.

3. A process according to claim 1, **characterized in that** R represents a methyl, ethyl or pentyl group or a phenyl group optionally substituted.

4. A process according to claim 1, **characterized in that** R¹, R², R³ and R⁴ represent, simultaneously or independently, a hydrogen atom, a methyl, ethyl or pentyl group or a phenyl group optionally substituted.

5. A process according to claim 1, **characterized in that** R, R¹, R² or R³ represents, simultaneously or independently, a methyl, ethyl or phenyl group optionally substituted, and R⁴ represents a hydrogen atom.

6. A process according to claim 1, **characterized in that** the enone (II) is obtained *in situ* by reacting together a ketone of formula 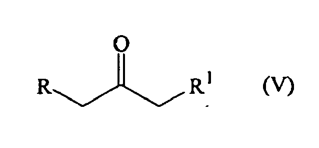 with an aldehyde or ketone of formula 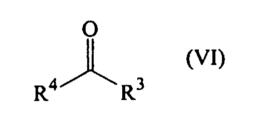 wherein R, R¹, R³ and R⁴ have the same meaning as in claim 1;
in the presence of a catalytic system as defined in claim 1.

7. A process according to claim 1, **characterized in that** M represents Ti(IV), R⁵ represents a linear or branched C₃₋₄ alkyl group, X represents a Cl atom and the index n represents 2 or 3

8. A process according to claim 5, **characterized in that** the co-ingredient of the catalytic system is selected from the group consisting of an alkyl or aromatic carboxylic acid anhydride containing 4 to 8 carbon atoms, BF₃, ClSi(R⁶)₃, R⁶ representing a C₁₋₅ alkyl group, and an anhydrous sulfate, chloride or bromide of a metal cation selected from the group consisting of Na⁺, K⁺, Mg²⁺, Ca²⁺, Zn²⁺, Fe³⁺.

## Patentansprüche

1. Verfahren für die Herstellung einer Verbindung der Formel 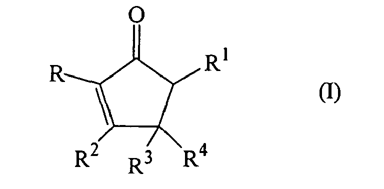 wobei R eine C₁₋₈-Alkyl- oder -Alkenylgruppe, gegebenenfalls substituiert, oder eine C₅₋₆-aromatische, gegebenenfalls substituiert, darstellt; und
R¹, R², R³ und R⁴, gleichzeitig oder unabhängig, ein Wasserstoffatom, eine C₁₋₈-Alkyl- oder -Alkenylgruppe, gegebenenfalls substituiert, oder eine C₅₋₆-aromatische Gruppe, gegebenenfalls substituiert, darstellen;
wobei das Verfahren die Umsetzung zwischen einem Enon der Formel 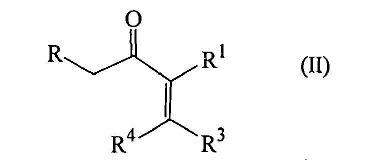 wobei R, R¹, R³ und R⁴ die gleiche Bedeutung wie in Formel (I) haben;
mit einem Aldehyd der Formel 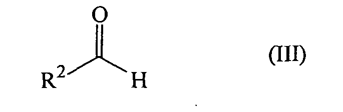 wobei R² die gleiche Bedeutung wie in Formel (I) hat, umfaßt; und
wobei die Umsetzung zwischen dem Enon (II) und dem Aldehyd (III) in Gegenwart eines katalytischen Systems durchgeführt wird, umfassend:
i) mindestens einen Metallkomplex der Formel
M(OR⁵)₄₋ₙXₙ (IV)
wobei M Ti(IV) oder Zr(IV) ist, R⁵ eine lineare oder verzweigte C₁₋₆-Alkylgruppe darstellt, X ein Halogenid darstellt und n eine ganze Zahl von 1 bis 3 darstellt; und
ii) mindestens einen Hilfsbestandteil, ausgewählt aus der Gruppe, bestehend aus
a) einem Alkyl- oder aromatischen Carbonsäureanhydrid, enthaltend 2 bis 10 Kohlenstoffatome;
b) einem wasserfreien Sulfat, Chlorid oder Bromid eines Metallkations, ausgewählt aus der Gruppe, bestehend aus Li⁺, Na⁺, K⁺, Cs⁺, Mg²⁺, Ni²⁺, Ca²⁺, Zn²⁺, Fe³⁺ und Al³⁺;
c) einem unlöslichen anorganischen Material, imstande, mit Wasser ein Clathrat zu bilden; und
d) einem C₄-C₁₅-Orthoester, BF₃, N-Methyl-N-trimethylsilyltrifluoracetamid, 1-Trimethylsilylimidazol und ClSi(R⁶)₃, wobei R⁶ eine C₁₋₅-Alkylgruppe darstellt.

2. Verfahren nach Anspruch 1, **dadurch gekennzeichnet, daß** R eine C₁₋₈-Alkyl- oder -Alkenylgruppe darstellt; und
R¹, R², R³ und R⁴, gleichzeitig oder unabhängig, ein Wasserstoffatom, eine C₁₋₈-Alkyl- oder -Alkenylgruppe darstellen.

3. Verfahren nach Anspruch 1, **dadurch gekennzeichnet, daß** R eine Methyl-, Ethyl- oder Pentylgruppe oder eine Phenylgruppe, gegebenenfalls substituiert, darstellt.

4. Verfahren nach Anspruch 1, **dadurch gekennzeichnet, daß** R¹, R², R³ und R⁴, gleichzeitig oder unabhängig, ein Wasserstoffatom, eine Methyl-, Ethyl- oder Pentylgruppe oder eine Phenylgruppe, gegebenenfalls substituiert, darstellen.

5. Verfahren nach Anspruch 1, **dadurch gekennzeichnet, daß** R, R¹, R² oder R³, gleichzeitig oder unabhängig, eine Methyl-, Ethyl- oder Phenylgruppe, gegebenenfalls substituiert, darstellen und R⁴ ein Wasserstoffatom darstellt.

6. Verfahren nach Anspruch 1, **dadurch gekennzeichnet, daß** das Enon (II) *in situ* erhalten wird, indem ein Keton der Formel 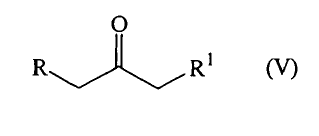 zusammen mit einem Aldehyd oder Keton der Formel 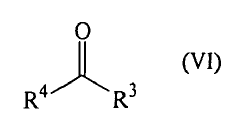 wobei R, R¹, R³ und R⁴ die gleiche Bedeutung wie in Anspruch 1 haben;
in Gegenwart eines katalytischen Systems, wie definiert in Anspruch 1, umgesetzt wird.

7. Verfahren nach Anspruch 1, **dadurch gekennzeichnet, daß** M Ti(IV) darstellt, R⁵ eine lineare oder verzweigte C₃₋₄-Alkylgruppe darstellt, X ein Cl-Atom darstellt und der Index n 2 oder 3 darstellt.

8. Verfahren nach Anspruch 5, **dadurch gekennzeichnet, daß** der Hilfsbestandteil des katalytischen Systems aus der Gruppe, bestehend aus einem Alkyl- oder aromatischen Carbonsäureanhydrid, enthaltend 4 bis 8 Kohlenstoffatome, BF₃, ClSi(R⁶)₃, wobei R⁶ eine C₁₋₅-Alkylgruppe darstellt, und einem wasserfreien Sulfat, Chlorid oder Bromid eines Metallkations, ausgewählt aus der Gruppe, bestehend aus Na⁺, K⁺, Mg²⁺, Ca²⁺, Zn²⁺, Fe³⁺, ausgewählt ist.

## Revendications

1. Procédé de préparation d'un composé de formule 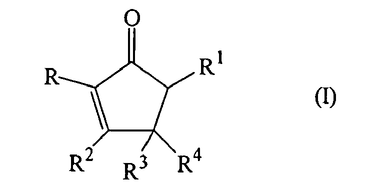 dans laquelle R représente un groupe alkyle ou alcényle en C₁₋₈ éventuellement substitué ou un groupe aromatique en C₅₋₆, éventuellement substitué; et
R¹, R², R³ et R⁴ représentent, simultanément ou indépendamment, un atome d'hydrogène, un groupe alkyle ou alcényle en C₁₋₈ éventuellement substitué ou un groupe aromatique en C₅₋₆, éventuellement substitué;
ledit procédé comprenant la réaction entre une énone de formule 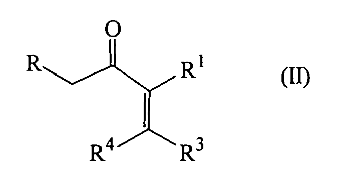 dans laquelle R, R¹, R³ et R⁴ ont la même signification que dans la formule (I);
avec un aldéhyde de formule 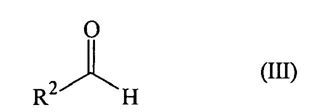 dans laquelle R² a la même signification que dans la formule (I); et
la réaction entre ladite énone (II) et l'aldéhyde (III) étant effectuée en présence d'un système catalytique comprenant:
i) au moins un complexe métallique de formule
M(OR⁵)₄₋ₙXₙ (IV)
dans laquelle M est Ti(IV) ou Zr(IV), R⁵ représente un groupe alkyle linéaire ou ramifié en C₁₋₆, X représente un halogénure et n représente un entier de 1 à 3; et
ii) au moins un co-ingrédient choisi dans le groupe consistant en
a) un anhydride d'acide carboxylique alkylique ou aromatique contenant 2 à 10 atomes de carbone;
b) un sulfate, chlorure ou bromure anhydre d'un cation métallique choisi dans le groupe consistant en Li⁺, Na⁺, K⁺, Cs⁺, Mg²⁺, Ni²⁺, Ca²⁺, Zn²⁺, Fe³⁺ et Al³⁺;
c) une matière inorganique insoluble capable de former un clathrate avec l'eau; et
d) un orthoester en C₄-C₁₅, BF₃, le N-méthyl-N-triméthylsilyl-trifluoroacétamide, le 1-triméthylsilylimidazole et ClSi(R⁶)₃, R⁶ représentant un groupe alkyle en C₁₋₅.

2. Procédé selon la revendication 1, **caractérisé en ce que** R représente un groupe alkyle ou alcényle en C_{1-8;} et
R¹, R², R³ et R⁴ représentent, simultanément ou indépendamment, un atome d'hydrogène, un groupe alkyle ou alcényle en C₁₋₈.

3. Procédé selon la revendication 1, **caractérisé en ce que** R représente un groupe méthyle, éthyle ou pentyle ou un groupe phényle éventuellement substitué.

4. Procédé selon la revendication 1, **caractérisé en ce que** R¹, R², R³ et R⁴ représentent, simultanément ou indépendamment, un atome d'hydrogène, un groupe méthyle, éthyle ou pentyle ou un groupe phényle éventuellement substitué.

5. Procédé selon la revendication 1, **caractérisé en ce que** R, R¹ , R² ou R³ représente, simultanément ou indépendamment, un groupe méthyle, éthyle ou phényle éventuellement substitué, et R⁴ représente un atome d'hydrogène.

6. Procédé selon la revendication 1, **caractérisé en ce que** l'énone (II) est obtenue *in situ* en faisant réagir ensemble une cétone de formule 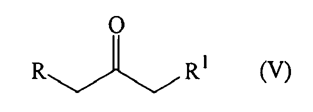 avec un aldéhyde ou une cétone de formule 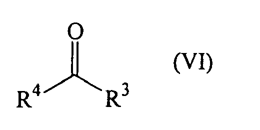 dans laquelle R, R¹, R³ et R⁴ ont la même signification que dans la revendication 1;
en présence d'un système catalytique tel que défini dans la revendication 1.

7. Procédé selon la revendication 1, **caractérisé en ce que** M représente Ti(IV), R⁵ représente un groupe alkyle en C₃₋₄ linéaire ou ramifié, X représente un atome de Cl et l'indice n représente 2 ou 3.

8. Procédé selon la revendication 5, **caractérisé en ce que** le co-ingrédient du système catalytique est choisi dans le groupe consistant en un anhydride d'acide carboxylique alkylique ou aromatique contenant 4 à 8 atomes de carbone, BF₃, ClSi(R⁶)₃, R⁶ représentant un groupe alkyle en C₁₋₅, et un sulfate, chlorure ou bromure anhydre d'un cation métallique choisi dans le groupe consistant en Na⁺, K⁺, Mg²⁺, Ca²⁺, Zn²⁺, Fe³⁺.
